# EUROPEAN PATENT SPECIFICATION

(11) **EP 3 103 351 B1**
(45) Date of publication and mention of the grant of the patent: **07.11.2018**
(21) Application number: 15742854.1
(22) Date of filing: 30.01.2015
(51) Int. Cl.: A23K 50/00, A23K 10/40

(54) **ORALLY ADMINISTERED AGENT FOR RUMINANTS AND RUMINANT FEED CONTAINING SAME**
ORAL VERABREICHTES MITTEL FÜR WIEDERKÄUER UND FUTTERMITTEL DAMIT
AGENT ADMINISTRÉ ORALEMENT POUR RUMINANTS ET ALIMENT POUR RUMINANTS CONTENANT CELUI-CI

(30) Priority: 31.01.2014 JP 2014017200
(43) Date of publication of application: 14.12.2016
(73) Proprietor: Morishita Jintan Co., Ltd., Osaka-shi Osaka 540-8566 (JP); Research Institute of Environment, Agriculture And Fisheries, Osaka Prefecture, Habikino-shi, Osaka 583-0862 (JP)
(72) Inventor: ADACHI, Takuhiko, Osaka-shi Osaka 540-8566 (JP); SEYAMA, Tomohiro, Habikino-shi Osaka 583-0862 (JP); HIRAYASU, Hirofumi, Habikino-shi Osaka 583-0862 (JP); KASAI, Koji, Habikino-shi Osaka 583-0862 (JP); FUJITANI, Yasuhiro, Habikino-shi Osaka 583-0862 (JP)
(74) Representative: dompatent von Kreisler Selting Werner - Partnerschaft von Patent- und Rechtsanwälten mbB
(86) International application number: PCT/JP2015/052754
(87) International publication number: WO 2015/115618

(56) References cited:
- WO-A1-02/082921
- JP-A- H0 531 352
- JP-A- S61 195 653
- JP-A- 2000 060 440
- JP-A- 2001 120 189
- US-A- 6 013 286
- US-A1- 2007 231 369

## Description

### TECHNICAL FIELD

The present invention relates to an orally administered agent for ruminants, particularly an orally administered agent which reaches lower digestive tract by avoiding the destruction thereof due to rumination, and ruminant feed containing it.

### BACKGROUND OF THE INVENTION

In a technical field of veterinary/animal husbandry, as part of the prevention and/or treatment for diseases of domestic ruminants such as cattle, administration of vitamin preparations and/or saccharide preparations has been performed. However, for example, in the cattle, there are ten billion to hundred billion of microorganisms per 1 g of stomach contents in a rumen, which is one of the four stomachs. Therefore, if vitamin B1 and/or saccharide preparations frequently used, for example, in a digestive disorder therapeutic and/or fast-acting nutritional supplementation are orally administered, microorganisms decompose them, and these components cannot be effectively absorbed and utilized in a body of the cattle. Thus, in order to effectively absorb and utilize these components in the body of the cattle, there is no way other than intravenous injection or subcutaneous injection thereof. However, these administration methods require expertise and expensive cost.

On the other hand, there is a possibility that the microorganisms held by cattle in its body may contaminate foods through processed meat and/or immature compost, etc. For example, food poisoning incident caused by O157, which is pathogenic E. coli derived from cattle, and the like has frequently occurred. There are cases when food poisoning incident may put patients to death if severe, and it is a major food risk in modern society.

In order to reduce the risk, development of a technique for preventing the discharge of pathogenic microorganisms which cause food poisoning by administering lactic acid bacteria to cattle has been attempted. For butyric acid bacteria and some of the lactic acid bacteria, effects of inhibiting growth of Shiga toxin producing E. coli have been confirmed by an experiment. However, the effects cannot be sufficiently accomplished only by administering lactic acid bacteria to the cattle from its mouth, because the lactic acid bacteria are diluted or killed in a rumen, and it has not yet become a practical technique for cattle. In order to solve this problem, the lactic acid bacteria must pass through four stomachs in its living state.

JP 2011-125217 A (Patent Document 1) discloses an additive composition for ruminant feed comprising at least one of protective agent selected from hydrogenated vegetable oil or hydrogenated animal oil having a melting point of 50 to 90°C, lecithin, acidic or neutral amino acid and water. In this technique, a mixture of oils and fats, lecithin and amino acids is molded by a granulator and then solidified in water to granulate it. The granulated product has rumen bypassing properties, and it is possible to promote milk production of lactating cows.

In the additive composition of Patent Document 1, the oils and fats are used as a protective agent such that the amino acid and the like are not decomposed in the rumen. In addition, a content rate of functional component is within the range of 40 to 60% by weight. However, the functional component leaks out in the rumen by destruction due to "rumination", that is, re-chewing the rumen contents. Therefore, it must be ingested in a large amount in order to obtain expected effects. The compositions are also requires complex processing methods depending on chemical properties of the individual functional substance, and the processing method itself has a low versatility. Therefore, it is required to develop new processing methods depending on the individual functional substance in order to develop new bypass oils and fats feed, which requires many time and many cost before it is developed.

JP 2009-159934 A (Patent Document 2) discloses a feed additive for providing to ruminants, wherein vitamin C is protected by primary encapsulating vitamin C with a binding coating agent, and further secondary encapsulating it with extremely cured oils and fats. Patent Document 2 discloses that the additive has excellent rumen bypassing rate. The primary encapsulated material is obtained by adding such fructose and/or propylene glycol to silica and hardened oils, the secondary encapsulated material is hardened palm stearin oil (See Examples in Patent Document 2). This technique is applied only to vitamin C, and it is difficult to apply to other functional components. In addition, there is a high risk of being destructed due to rumination by re-chewing.

JP 2009-535056 A (Patent Document 3) discloses a method of preparing rumen protected choline in order to supply choline (substance for water-soluble vitamin) to lactating cows, wherein the method comprises the steps of carrying out the choline on a carrier, coating with hydrogenated oil, further selecting a material having small particle size therefrom, and doubly coating with oils and fats. This technique is also applied only to choline, and it is difficult to apply to other substances. In addition, there is a high risk of being destructed due to rumination by re-chewing.

JP 2001-120189 A (Patent Document 4) discloses a composition for providing fatty acid to ruminants. When orally administering fatty acid calcium from fish oil, such as bonito and tuna, to ruminants by using the composition, it is possible to produce milk and meat richly containing DHA or EPA in fish oil by heightening a specific gravity of the fatty acid calcium from fish oil and by adjusting the particle size to not more than 3 mm. This technique is also applied only to the fish oil, and it is difficult to apply to other substances. In addition, the specific gravity and particle size of the composition are limited, but the breaking strength is not examined.

### PRIOR ART

### Patent Documents

Patent Document 1: JP 2011-125217 A
Patent Document 2: JP 2009-159934 A
Patent Document 3: JP 2009-535056 A
Patent Document 4: JP 2001-120189 A

US 6,013,286 discloses a composition and method for administering a bioactive substance to the post-rumen portion of the digestive system of a ruminant substantially without introducing the substance to the rumen portion of the digestive system. The composition of matter comprises particles which have a specific gravity between 0.3 and 2.0, preferably between 1.0 and 2.0. The particles comprise a core comprising bioactive substance; a hydrophobic coating essentially completely encapsulating said. core in a quantity sufficient to essentially preclude introduction of bioactive substance into the rumen; and a surfactant applied to the surface of the hydrophobic coating in a quantity sufficient to ensure that said particles do not float on the rumen. The method comprises orally administering this composition of matter to a ruminant.

US 2007/231369 discloses a ruminant feed composition comprising a core material of at least one biologically active substance coated with a polymer coating formed in situ on the core material, and an outer intermediate wax coating surrounding the polymer coating, wherein said polymer coating and intermediate wax coating provides for effective non-release of the biologically active substance in a rumen of a ruminant animal.

WO 02/082921 discloses a rumen bypass composition containing a bioactive substance capable of releasing said bioactive substance into the post rumen portion of the digestive system of a ruminant without introducing into the rumen of the digestive system. The composition is in the form of beadlets and comprises of a core consisting of a bioactive substance; a hydrophobic coating material, which completely encapsulates the core to avoid interaction within the rumen of a ruminant; an adjuvant hydrophobic in nature having a melting range 50 to 80 °C added to the coating material to improve thermal resistance wherein said beadlets have a size of 2 to 8 mm, the amount of said bioactive material being in the range of 30 to 60 % of the weight of the beadlet, the amount of said coating material. is in the range of from 40 to 80 % by weight and the amount of said adjuvant being in the range of from 5 to 50 % based on the weight of said coating material. The method preparation comprises mixing an aqueous bioactive substance with a coating material, heating the mixture under constant stirring at a temperature in the range of 55 to 80 °C to produce a molten mass, allowing drops of said molten mass to fall from a height through pre-cooled air over a cold surface to obtain said composition in the form of beadlets.

JP H05-31352 discloses a seamless capsule consisting of a content and a film coating the content, wherein the content is a hydrophilic substance dissolved in a solvent having a high hygroscopicity. A viscous liquid that can hardly be mixed with water and having viscosity of 1000 cp or less at 100 °C is present between the content and the film.

JP 2000-060440discloses a feed additive composition for ruminant having protective property in the rumen and dissolution property in the abomasum. In particular, it discloses granules that are formed by homogeneously mixing (a) amino acids, (b) an oily binding agent having a melting point of 40 °C or more and (c) water needed for extrusion granulation, and subjecting the obtained mixture to the extrusion granulation at the melting point of said oily binding agent; or by homogeneously mixing (a) amino acids and (b) an oily binding agent having a melting point of 40 °C or more, feeding the obtained homogeneous powdery mixture into an extrusion granulator and granulating it at the melting point of said oily binding agent while spraying (c) water before a die. The obtained granules are dried, and if needed or optionally, the surfaces of the dried granules are coated with an oil-and-fat coating agent to produce granules having a percentage of void of 20 % or less.

### OBJECTS OF THE INVENTION

The present invention provides an orally administered agent for ruminants which reaches lower digestive tract without decomposition in the rumen by avoiding the destruction thereof due to rumination, and is disintegrated and dissolved in vivo.

### SUMMARY OF THE INVENTION

In order to solve the problems, the present invention provide the following embodiments:
[1] An orally administered agent for ruminants which reaches lower digestive tract without decomposition in the rumen, wherein a specific gravity determined using isooctane as a specific gravity standard solution is within the range of 1.17 to 2.00, a maximum particle size is within the range of 4 to 10 mm and a minimum particle size is within the range of 4 to 10 mm, and a breaking strength of a portion having the minimum particle size under body temperature environment of the ruminants is within the range of 0.5 to 5.0 N.
[2] The orally administered agent for ruminants, wherein the orally administered agent is a seamless capsule comprising a core, and at least one layer of shell film layer covering the core, wherein the core and shell film layer either comprise a specific gravity regulator, and wherein the core further comprises a carrier and a drug for ruminants.
[3] The orally administered agent for ruminants, wherein the shell film layer is comprised of two layers of an inner shell film in direct contact with the core and an outer shell film as an outermost layer,
   wherein the core further comprises a specific gravity regulator,
   wherein the inner shell film comprises a specific gravity regulator, and an oily substance having a melting point of 45 to 90°C, and
   wherein the outer shell film is formed from a shell film composition comprising polysaccharide, a specific gravity regulator, and a plasticizer.
[4] The orally administered agent for ruminants, wherein the core has a melting point of 32 to 44°C.
[5] The orally administered agent for ruminants, wherein the drug for ruminants is selected from the group consisting of herbal medicine extract, tincture, a therapeutic agent, plant extract, animal extract, microorganism extract, microbial production extract, fruit juice, functional polysaccharides, polyphenols, vitamin C, vitamin B, amino acids, microorganisms, bacteria, essential oil, ω-3 fatty acid, ω-6 fatty acid, ω-9 fatty acid and combinations thereof.
[6] A method, of making the above-mentioned orally administered agent for ruminants comprising the steps of:
   by using a first nozzle, a second nozzle and a third nozzle such that they are concentrically arranged and have a radius sequentially increased from the first nozzle to the third nozzle, ejecting a core preparation composition through the first nozzle, ejecting an inner shell film preparation composition through the second nozzle and ejecting a shell film composition through the third nozzle simultaneously to form a composite jet, and
   releasing the composite jet into an oil solution,
   wherein the core preparation composition comprises a specific gravity regulator, a carrier and a drug for ruminants,
   wherein the inner shell film preparation composition comprises a specific gravity regulator, and an oily substance having a melting point of 45 to 90°C, and
   wherein the shell film composition comprises polysaccharide, a specific gravity regulator, and a plasticizer.
[7] A ruminant feed comprising the orally administered agent for ruminants. The orally administered agent may be orally administered to the ruminants.

### EFFECTS OF THE INVENTION

The present inventors prepared particulates having various specific gravity and various particle size, and orally administered the particulates to ruminants. As the results, the present inventors found by an experiment that there were the values of the specific gravity and particle size such that a large amount of the particulates were excreted from the ruminants in the particulates orally administered. Based on the experimental results obtained as described above, particulates having various breaking strength (a breaking strength of a portion having the minimum particle size) were further prepared and were orally administered to ruminants, and an optimum value of the breaking strength was selected by measuring a blood concentration of a drug contained in the particulates. Based on these experiments, the specific gravity, particle size, and breaking strength which are optimal as an orally administered agent for ruminants have been found. Then, by preparing an orally administered agent which satisfy conditions experimentally found, it has been confirmed by an experiment that the functional component is not decomposed in the rumen of ruminants, passes through the rumen to abomasum to reach lower digestive tract in and after intestine, and the orally administered agent is disintegrated by peristaltic motion.

That is, in the present invention, the specific gravity, particle size, and breaking strength which are optimal as an orally administered agent for ruminants have been found. When the functional component is enclosed in the orally administered agent satisfying these conditions and it is orally administered to ruminants, the functional component can be validly and effectively ingested by ruminants.

When using the orally administered agent of the present invention, drugs for ruminants, which could be previously administered only by a method such as injection, may reach lower digestive tract and be ingested merely by adding it, for example, to ruminant feed. Therefore, the administration and ingestion of the drug to ruminants becomes very easy as compared with conventional methods.

Furthermore, according to the orally administered agent of the present invention, it is possible to administer a drug to ruminants by merely changing a type of the drug to be included in a core of the orally administered agent. Therefore, it is possible to easily prepare the orally administered agent depending on a variety of drugs, and processability and versatility is very high, thereby there is an advantage that it is possible to be applied to various types of drugs.

### BRIEF DESCRIPTION OF THE DRAWINGS

Fig.1 to Fig.4 are graphs illustrating cumulative excretion rate of beads having each diameter in case of administering beads of a different type of resin, that is, beads having a different specific gravity to cattle.
Fig.1 is a graph illustrating data of high-density polyethylene (PE) beads.
Fig.2 is a graph illustrating data of poly(methyl methacrylate) (PMMA) beads.
Fig.3 is a graph illustrating data of polyoxymethylene (POM) beads.
Fig.4 is a graph illustrating data of polytetrafluoroethylene (PTFE) beads.
Fig.5 is a graph illustrating comparison of four types of resins of beads having a particle size of 6 mm, that is, comparative data with respect to specific gravity.
Fig.6 is a graph illustrating time-dependent change of blood thiamine concentration, when capsules containing vitamin B1 of Examples 2 to 7 and Comparative Examples 1 and 2 are orally administered to six adult cattle in a forced manner by using an oral administration device. As a control, a graph illustrating time-dependent change of blood thiamine concentration, when aqueous solution of vitamin B1 is orally administered, is also shown.
Fig.7 is a graph illustrating cumulative excretion of urinary ascorbic acid, when Vitamin C30% Bypass (YPTECH Co., Ltd.) and the capsule of Example 8 are orally administered to six adult cattle in an amount such that vitamin C is 30 g in a forced manner by using an oral administration device.

### DESCRIPTION OF THE PREFERRED EMBODIMENTS

In the present invention, it has been found by experiment that there is a resin sphere such that it is the most hardly ruminated and it rapidly reaches lower digestive tract by administering resin spheres having a different specific gravity and/or particle size to livestock such as cattle, and measuring a passage rate thereof. Based on the experimental results, it has been confirmed by experiment that there are values of a specific gravity and particle size effective as an orally administered agent for ruminants. The particle size specifically means both the maximum particle size and the minimum particle size. The orally administered agent of the present invention may have a variety of shapes, but the both maximum particle size and minimum particle size must be within a given range.

Next, in the present invention, an arrival and absorption of a drug to intestinal tract have been verified by enclosing the drug in capsules having a different breaking strength of a portion having the minimum particle size and measuring time-dependent change of the drug in blood after administration to livestock such as cattle as compared with a case of orally administering the drug without enclosing in a capsule. By this experiment, the breaking strength of a portion having the minimum particle size such that it can be the most effectively reflected to a blood concentration was found. As the reason for measuring the breaking strength of a portion having the minimum particle size of the capsule, this is because the construction of a mechanism that a drug included in the capsule is released by the destruction of the capsule due to peristaltic motion of the lower digestive tract is on the basis of the minimum particle size such that the breaking strength of the capsule is the lowest. Incidentally, the breaking strength in the present invention refers to a breaking strength under the environment of 39°C, which is a body temperature of cattle.

It has been found from these experiments that in the orally administered agent for ruminants of the present invention, it is required that a specific gravity is within the range of 1.17 to 2.00, a maximum particle size is within the range of 4 to 10 mm and a minimum particle size is within the range of 4 to 10 mm, and a breaking strength of a portion having the minimum particle size is within the range of 0.5 to 5.0 N. The specific gravity literally means a specific gravity of the entire orally administered agent, which is determined by dividing a weight of the orally administered agent by a volume thereof. The specific gravity of the orally administered agent of the present invention is within the range of preferably 1.20 to 1.70, more preferably 1.25 to 1.45. When the specific gravity is lower than 1.17, there is a low probability that the orally administered agent is excreted in an intact state when administered to ruminants. Since the specific gravity is low, the orally administered agent floats in the ruminant stomach, and this is probably due to the fact that it is many times exposed to rumination. On the other hand, when the specific gravity is higher than 2.00, excretion efficiency is reduced or the orally administered agent is not excreted when administered to ruminants. Since the specific gravity is high, the orally administered agent is settled in the ruminant stomach, and this is probably due to the fact that it is difficult to perform active transport into the lower digestive tract than the ruminant stomach.

The orally administered agent of the present invention is required that the maximum particle size is within the range of 4 to 10 mm and a minimum particle size is within the range of 4 to 10 mm. Both of the maximum particle size and minimum particle size are within the range of preferably 5 to 9 mm, more preferably 6 to 8 mm. The orally administered agent may have any shape (particulate), but it is preferable to have a spherical shape because a drug for ruminants is enclosed therein and an interior volume is maximized. If it has a spherical shape, the maximum particle size and minimum particle size are approximately the same value as each other, which are a diameter of sphere. The orally administered agent of the present invention may have a cubic shape, rectangular parallelepiped shape, conical shape, cylindrical shape and the like, but both of the maximum particle size and minimum particle size must be within the above range.

As used herein, the maximum particle size and minimum particle size of the orally administered agent are values measured by performing digital processing using a microscope such as a digital microscope. Examples of the digital microscope include, for example, VHX series (such as the VHX-2000, VHX-5000) from KEYENCE Corporation.

The orally administered agent of the present invention has a breaking strength of a portion having the minimum particle size of 0.5 to 5.0 N. The breaking strength is within the range of preferably 0.6 to 4.8 N, more preferably 0.7 to 4.7 N. The breaking strength of a portion having the minimum particle size represents easiness of disintegrating the orally administered agent, and can be determined using a normal physical property measurement device such as a comprehensive physical property measurement device (Sun Scientific Co., Ltd, rheometer) by pressing a circular plunger having a diameter of 10 mm against the orally administered agent in the minimum particle size direction (at a table speed of 20 mm/min), and measuring a load required to break the orally administered agent. When the breaking strength of a portion having the minimum particle size is lower than 0.5 N, the orally administered agent is easily disintegrated by chewing of ruminants; or is disintegrated before reaching the lower digestive tract by convection caused in the rumen and reticulum during rumination or stress caused when actively transporting into the lower digestive tract than the rumen; and a drug enclosed in the orally administered agent is leaked out and is decomposed by microorganisms in the rumen. On the other hand, when the breaking strength of a portion having the minimum particle size is higher than 5.0 N, the orally administered agent is not disintegrated by peristaltic motion in the lower digestive tract of ruminants and is excreted in an intact state, and it is not possible to release the drug into the body of ruminants.

In the present invention, if the orally administered agent satisfies the specific gravity, the maximum particle size, the minimum particle size, and the breaking strength of a portion having the minimum particle size, it reaches the lower digestive tract of ruminants from the stomach including the rumen in the ruminants without much damage, and it is easy to administer a drug enclosed in the orally administered agent to the ruminants. The orally administered agent may be a tablet, a pill, a granule or a capsule, but a spherical capsule, specifically a seamless capsule is particularly preferable because an interior volume may be maximized.

Hereinafter, it will be described in detail for the case that the orally administered agent is a seamless capsule. It is desired that the orally administered agent of the present invention is a seamless capsule comprising a core, and at least one layer of shell film layer covering the core; and a carrier and a drug for ruminants are contained in a composition if the core.

The shell film layer of the seamless capsule is comprised of one or more shell film layers. The shell film layer preferably has a two-layered structure, and is preferably comprised of an inner shell film in direct contact with the core and an outer shell film as an outermost layer. The inner shell film preferably comprises a specific gravity regulator, and an oily substance having a melting point of 45 to 90°C. In addition, the outer shell film is preferably formed from a shell film composition comprising polysaccharide, a specific gravity regulator, and a plasticizer.

### Core

The core comprises a carrier and a drug. The carrier used for the core is not particularly limited as long as it is a substance to dilute, hold and support the drug for ruminants without decomposing the drug. The carrier preferably has a melting point of 10 to 45°C, more preferably 15 to 40°C, and may be a hydrophilic solvent or non-hydrophilic solvent. The carrier is preferably a liquid during producing the seamless capsules in a point of view of production. In addition, the carrier is preferably a solid during storing or orally administering the seamless capsule in a point of view of storability. A type of the substance used for the carrier may be suitably set depending on a production temperature and storage temperature. As a carrier, for example, a substance such that it is a liquid at 15 to 45°C, preferably 15 to 44°C, more preferably 20 to 44°C is suitably used. On the other hand, as a carrier for an orally administered agent used in a cold district, a substance such that it is a liquid at 4 to 20°C is suitably used. In addition, as a carrier for an orally administered agent used in tropic regions, a substance such that it is a liquid at 25 to 60°C is suitably used.

Examples of the carriers include, for example, oils and fats and derivatives thereof, fatty acid esters, hydrocarbons (such as aliphatic hydrocarbons, aromatic hydrocarbons), ethers, higher alcohols, terpenes, sterols, silicones, beeswax and derivatives thereof, and the like phospholipids. These substances may be used alone, or may be used in combination of two or more thereof.

Examples of the oils and fats and their derivatives include soybean oil, rice oil, sesame oil, palm oil, palm kernel oil, corn oil, peanut oil, cottonseed oil, coconut oil, rapeseed oil, olive oil, cacao butter, beef tallow, lard, horse oil, whale oil, margarine, shortening and hydrogenated oils thereof.

Examples of the fatty acid esters include glycerin fatty acid esters (such as fatty acid monoglyceride, fatty acid diglyceride, fatty acid triglyceride), fatty acid esters of sugars (such as sucrose fatty acid esters, sorbitan fatty acid esters). As the fatty acid used in the fatty acid esters or fatty acid esters of sugars, medium-chain fatty acids (specifically, fatty acids having 8 to 12 carbon atoms) and long chain fatty acids (specifically, fatty acids having 14 to 18 carbon atoms) are preferably used, but are not limited thereto.

Examples of the hydrocarbons include aliphatic hydrocarbons, such as petroleum ether, pentane, hexane, heptane, octane, and derivatives thereof (such as haloalkanes); and aromatic hydrocarbons, such as benzene, toluene, xylene, and derivatives thereof.

Examples of the ethers include dipropyl ether, ethyl t-butyl ether.

Examples of the higher alcohols include decyl alcohol, dodecyl alcohol, myristyl alcohol, cetyl alcohol.

Examples of the terpenes include camphor oil, peppermint oil, α-pinene, D-limonene and the like.

Among the carriers, particularly, derivatives of the fats and oils (particularly hydrogenated oils of the fats and oils), medium chain fatty acids or long chain such as triglycerides or diglycerides of medium chain fatty acids or long chain fatty acids, and the like are suitably used in a point of view of storage stability of a drug included for ruminants and easy formulation.

The core, if necessary, may further include emulsifying agents, such as glycerin fatty acid esters, polyglycerol fatty acid esters, glycerin succinic acid fatty acid esters, sucrose fatty acid esters, sorbitan fatty acid esters, lecithin. These emulsifying agents may be used alone, or may be used in combination of two or more thereof.

The drug for ruminants enclosed in the core may be any drug, as long as it provides an improvement effect on growth, illness, nourishing, intestinal regulation and the like of ruminants. The drug may be either water soluble substances or water insoluble substances. Examples of the drugs for ruminants include, for example, medicinal extracts, such as Kakkonto extract, Shosaikoto extract; tinctures, such as bitter tincture, Saussurea Root tincture; acetaminophen, mexiletine hydrochloride, acarbose, cromolyn sodium, pravastatin sodium.

Further examples of the drugs for the ruminants of the present invention include, for example, plant extracts such as plum pulp extract, Momordica grosvenori extract, pomegranate extract, blueberry extract; animal extracts such as freshwater clam extract; microbial extracts such as yeast extract; microbial production substance; fruit juice such as lemon juice, apple juice, grape juice, peach juice; functional polysaccharides such as mucopolysaccharide; chlorella; peptide; polyphenols; vitamin C; vitamin B group; amino acid; useful microorganisms or bacteria, such as lactic acid bacteria, yeast, photosynthetic bacteria, actinomycetes; essential oil obtained from sassafras, clove, sage, Eucalyptus, damask rose, Mayorama, cinnamon, lemon, lime, grapefruit, and orange; ω-3 fatty acids, such as α-linolenic acid, stearidonic acid, eicosatrienoic acid , eicosatetraenoic acid, eicosapentaenoic acid, docosapentaenoic acid, docosahexaenoic acid, tetracosapentaenoic acid, tetracosahexaenoic acid; ω-6 fatty acids such as linoleic acid, γ-linolenic acid, eicosadienoic acid, dihomo-γ-linolenic acid, arachidonic acid, docosadienoic acid, docosatetraenoic acid, docosapentaenoic acid, calendic acid and the like; ω-9 fatty acids, such as oleic acid.

The core is formed by mixing the carrier and the drug for ruminants. A weight ratio of the drug to carrier is not more than 400 parts by weight, preferably 100 parts by weight of the drug, based on 100 parts by weight of the carrier. When the amount of the drug is larger than 400 parts by weight, fluidity of the core preparation composition during producing the seamless capsule is reduced, and there is a possibility that it is difficult to produce the seamless capsule.

The core constituting the orally administered agent of the present invention optionally may contain a specific gravity regulator for the purpose of adjusting the specific gravity of the capsule. The specific gravity regulator is preferably a pigment, more preferably an inorganic pigment, lake pigment, inorganic phosphor, particularly preferably inorganic pigments such as titanium dioxide, zinc oxide, ferric oxide (iron sesquioxide), silicon dioxide, calcium carbonate, talc, and mica, but it is not limited thereto. The specific gravity regulator may be used alone or may be used in combination of two or more thereof. If using the specific gravity regulator, the content thereof is within the range of preferably 0.1 to 60% by weight, more preferably 0.1 to 50% by weight, based on total solid content of the core.

In the orally administered agent of the present invention, the core has a melting point of preferably 32 to 44°C, more preferably 34 to 42°C. When the melting point of the core is lower than 32°C, the breaking strength of a portion having the minimum particle size of the orally administered agent is less than 0.5 N, if administered to ruminants, the capsule is disintegrated before reaching the lower digestive tract by convection caused in the rumen and reticulum during rumination or stress caused when actively transporting into the lower digestive tract than the rumen, and a drug enclosed in the capsule is leaked out and there is a low possibility that the drug is reflected to a blood concentration. On the other hand, when the melting point of the core is higher than 44°C, the breaking strength of a portion having the minimum particle size of the orally administered agent is higher than 5.0 N, if administered to ruminants, the capsule is not disintegrated by peristaltic motion in the lower digestive tract of ruminants, and there is a possibility that the capsule is excreted along with feces while the drug enclosed in the capsule is not reflected to a blood concentration.

### Inner shell film

The orally administered agent of the present invention preferably has an inner shell film between a core and an outer shell film which is the outermost layer. The inner shell film is preferably comprised of an oily substance having a melting point of 45 to 90°C. The inner shell film may further include an optional specific gravity regulator. The oily substance constituting the inner shell film has a melting point of more preferably 45 to 80°C, further more preferably 50 to 70°C. In the orally administered agent of the present invention, it is possible to suitably design the resulting breaking strength of the orally administered agent within the range of 0.5 to 5.0 N by providing an inner shell film comprised of the oily substance between the core and the outer shell film. Examples of the oily substances include, for example, fats and oils, fatty acids, waxes, fatty acid esters, higher alcohols, sterols, silicones, paraffins, beeswax, phospholipids, and derivatives thereof (such as hydrogenated oils or partially hydrogenated oils, etc.), which have a melting point of 45 to 90°C. These substances may be used alone, or may be used in combination of two or more thereof. Among these, one or more members selected from the group consisting of fats and oils and derivatives thereof, fatty acids and waxes, are preferably used as an oily substance.

Examples of the fats and oils and derivatives thereof include, for example, cacao butter, beef tallow, lard, horse oil, whale oil, margarine, shortening, rice hardened oil (such as rice extremely hardened oil, and rice and a half hardened oil, etc.), hydrogenated castor oil, hydrogenated rapeseed oil, hydrogenated palm oil, hydrogenated palm kernel oil, hydrogenated fish oil.

Examples of the fatty acids include, for example, palmitic acid, stearic acid, myristic acid and the like.

Examples of the waxes include, for example, rice wax, carnauba wax, candelilla wax, paraffin wax.

The fats and oils described in the above carrier, such as fatty acid esters or higher alcohols, may be optionally mixed with the oily substance. However, it is necessary to use the fats and oils in an amount such that a melting point of the inner shell film is within the range of 45 to 90°C.

Optionally, the inner shell film may further comprise emulsifying agents, such as glycerin fatty acid esters, polyglycerol fatty acid esters, sucrose fatty acid esters, sorbitan fatty acid esters, lecithin and the like. These emulsifying agents may be used alone, or may be used in combination of two or more thereof.

The inner shell film may optionally comprise a specific gravity regulator. As the specific gravity regulator, those described in the explanation of the core are suitably used. If using the specific gravity regulator, the content thereof is within the range of preferably 0.1 to 60% by weight, more preferably 0.1 to 50% by weight, based on total solid content of the inner shell film.

### Outer shell film

As the outer shell film, those used in normal as the outermost layer of the seamless capsule may be used. Examples of the outer shell films include, for example, an outer shell film formed from a shell film composition containing polysaccharides, a specific gravity regulator and a plasticizer.

Examples of the Polysaccharides, which are not limited thereto, include, for example, dextrin, starch, agar, carrageenan, gum arabic, gellan gum, xanthan gum, pectin, alginic acid and derivatives thereof. The polysaccharides are components forming a shell film. The content of the polysaccharides is within the range of preferably 50 to 95% by weight, more preferably 50 to 90% by weight, based on the total solid content of the shell film composition of the capsule.

The plasticizers are generally used for the purpose of changing the properties of the resulting shell film. Examples of the plasticizers, which are suitably used, include polyhydric alcohols, such as glycerin, ethylene glycol, polyethylene glycol, propylene glycol, polypropylene glycol; sugar alcohols, such as maltitol, mannitol, sorbitol, erythritol; or trehalose. The plasticizers may be used alone, or may be used in combination of two or more thereof. If using the s plasticizer, the content thereof is within the range of preferably 1 to 40% by weight, more preferably 5 to 30% by weight, based on total solid content of the shell film composition of the capsule.

The specific gravity regulator is used for the purpose of adjusting the specific gravity of the capsule. The specific gravity regulator is preferably a pigment, more preferably an inorganic pigment, lake pigment, inorganic phosphor, particularly preferably inorganic pigments such as titanium dioxide, zinc oxide, ferric oxide (iron sesquioxide), silicon dioxide, calcium carbonate, talc, and mica, but it is not limited thereto. The specific gravity regulator may be used alone or may be used in combination of two or more thereof. The specific gravity regulator may be used in the content within the range of preferably 0.1 to 60% by weight, more preferably 0.1 to 50% by weight, based on total solid content of the shell film composition of the capsule.

The outer shell film of the present invention may optionally comprise various additives usually used in this technical field, which include perfumes, sweetening agents, coloring agents, preservatives such as paraben, in addition to the above composition. If using such additives, the total content of all the additives is within the range of, for example, 0.01 to 10% by weight, preferably 0.1 to 5% by weight, based on the total solid content of the shell film composition of the capsule.

### Seamless capsule

The orally administered agent for ruminants of the present invention is preferably a seamless capsule. Examples of methods of producing the seamless capsule include, for example, a method of continuously producing a seamless capsule by a dropping method using a multiple nozzle, such as methods described in, for example, JP 58-22062 A, JP 59-131355 A, JP 3-52639 A, JP 5-031352 A, JP 7-069867 A, but it is not necessarily limited to the methods.

The seamless capsules may be produced, for example, by dropping method using a multiple nozzle. As the multiple nozzle, a concentric multiple nozzle having double or more nozzle may be used, and a concentric multiple nozzle having a triple nozzle may be suitably used. In a specific example of the dropping method using the concentric multiple nozzle having a triple nozzle, seamless capsules may be continuously produced due to interfacial tension acting between the oil solution and the shell film composition by ejecting a core preparation composition for preparing a core from an innermost nozzle of a concentric multiple nozzle having a triple nozzle, ejecting an inner shell film preparation composition containing an oily substance from an intermediate nozzle and ejecting the shell film composition from an outermost nozzle simultaneously at a constant rate into an oil solution flowing down at a steady speed to form a composite jet; and releasing the jet stream into an oil solution.

In this production method, it is preferable that the core preparation composition comprises a specific gravity regulator, a carrier and a drug for ruminants; the inner shell film preparation composition comprises a specific gravity regulator and an oily substance having a melting point 45 to 90°C; and the shell film composition comprises polysaccharide, a specific gravity regulator and a plasticizer.

In the production method, it is more preferable to heat the concentric multiple nozzle for releasing the core preparation composition, inner shell film preparation composition and shell film composition to a temperature such that the core preparation composition, inner shell film preparation composition and shell film composition have a viscosity of 10 to 300mPa·s. This heating temperature is, for example, preferably 10 to 90°C, more preferably 35 to 90°C.

In such a seamless capsule, the outer shell film layer preferably has a thickness of 30 to 400 µm.

In the seamless capsule as the orally administered agent for ruminants of the present invention, the specific gravity, particle size and breaking strength of a portion having the minimum particle size must be adjusted to specified ranges. For example, in the case of seamless capsule of a three-layered structure comprised of a core, an inner shell film and an outer shell film, the specific gravity may be adjusted by adding a specific gravity regulator for increasing a weight to at least one layer among the three layers. As the specific gravity regulator, it is possible to suitably use those described in the explanation of the core. For example, it is possible to adjust the specific gravity of the resulting seamless capsules by adding the specific gravity regulator to at least one of the core preparation composition, inner shell film preparation composition and shell film composition. Here, an embodiment of the seamless capsule of the present invention, of which the specific gravity is adjusted by adding the specific gravity regulator to all of the core preparation composition, inner shell film preparation composition and shell film composition, is more preferable.

In the case of forming the seamless capsule by a dropping method, the particle size may be easily adjusted by adjusting a flow rate of a cooling liquid and an extrusion amount from a nozzle.

The breaking strength of the seamless capsule may be adjusted, for example, by suitably selecting a component and content of the shell film composition for forming the outer shell film. In addition, it is possible to increase the breaking strength by selecting an each component such that the melting point is within relatively high range in the core and the oily substance of the inner shell film. Furthermore, it is possible to reduce the breaking strength by selecting an each component such that the melting point is within relatively low range. It is possible to obtain a breaking strength suitable for using as an orally administered agent for ruminants by adjusting the melting point of the core to the range of 32 to 44°C and using an oily substance having a melting point of 45 to 90°C as the oily substance of the inner shell film in the above selection.

### EXAMPLES

The present invention is explained in more detail based on the following Examples and Comparative Examples. In the examples, parts and percentages are based on weight unless otherwise indicated.

### Example 1

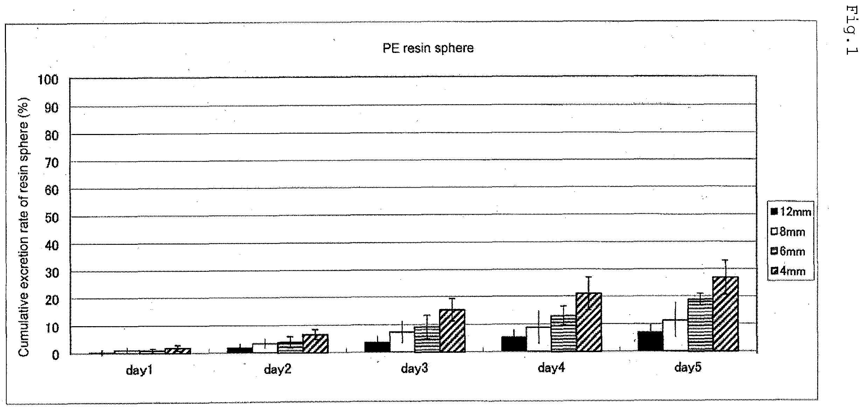

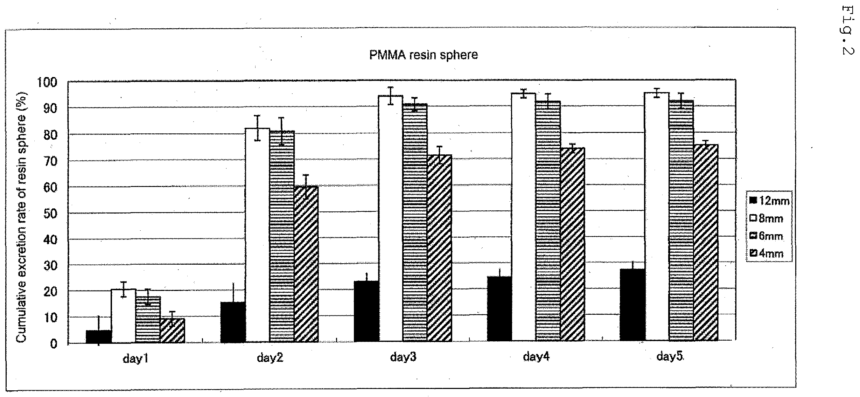

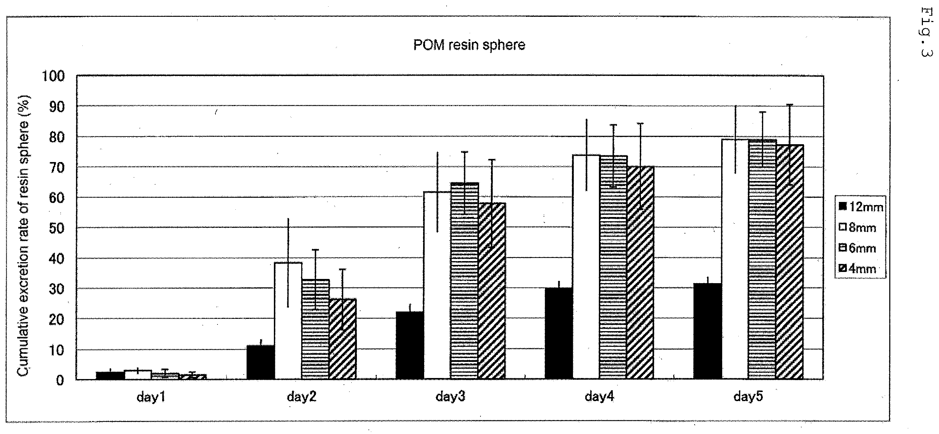

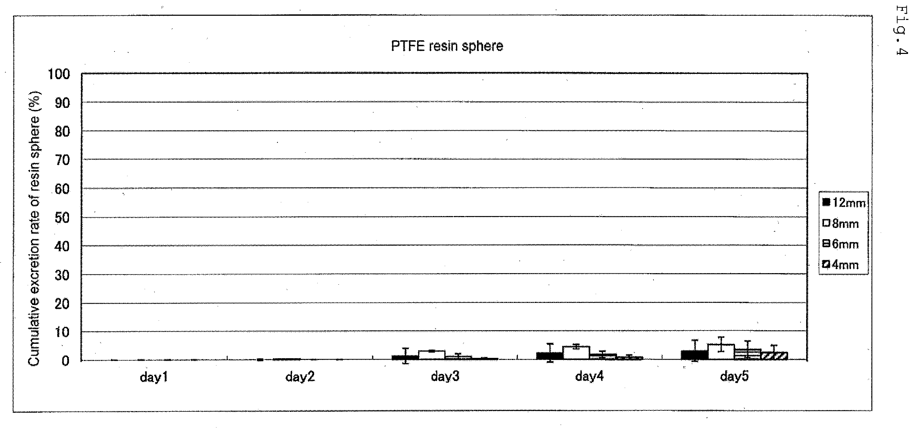

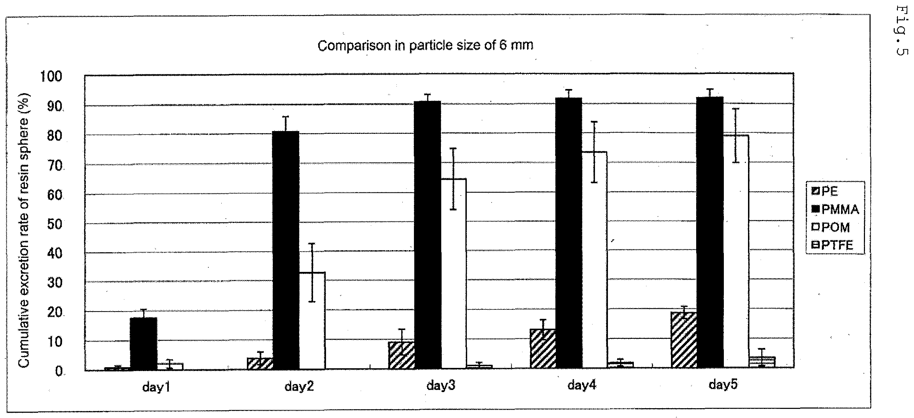

Every one hundred commercially available beads of high density polyethylene (PE) (specific gravity 0.95), poly(methyl methacrylate) (PMMA) (specific gravity 1.19), polyoxymethylene (POM) (specific gravity 1.41), and polytetrafluoroethylene (PTFE) (specific gravity 2.20) with respect to each particle size of 4 mm, 6 mm, 8 mm and 12 mm, respectively, were orally administered to four lactating cows. Cumulative rate of the number of beads in an intact state, which were excreted in feces, were described in Fig.1 to Fig.5. Fig.1 to Fig.4 showed a cumulative excretion rate with respect to beads of a different type of resin, that is, a cumulative excretion rate in every diameter with respect to beads having a different specific gravity. In addition, FIG. 1 is data of high-density polyethylene (PE) beads, Fig.2 is data of poly(methyl methacrylate) (PMMA) beads, Fig.3 is data of polyoxymethylene (POM) beads, and Fig.4 is data of polytetrafluoroethylene (PTFE) beads. Figure 5 is comparison of four types of resins in the beads having a particle size of 6 mm, that is, comparative data by the specific gravity.

Here, the diameter of these beads was determined by performing digital processing with a digital microscope VHX-2000 (KEYENCE Corporation).

As a result, the beads of poly(methyl methacrylate) (PMMA) having a diameter of 6 mm shows the highest cumulative excretion rate, which it is understood that it is higher than 90%. Further, the cumulative excretion rate of the beads having a particle size of 6 mm and 8 mm is higher than that of the beads having a particle size of 4 mm and 12 mm. The cumulative excretion rate of the beads having a specific gravity of 0.95 and 2.20 is poor and the beads having a specific gravity of 1.19 and 1.41 are excreted in higher rate.

### Example 2

### Production of seamless capsule

In a warmed solution of 60 parts of Melano H1000S (Hydrogenated palm kernel oil having a melting point 34°C (32 to 36°C) manufactured by Fuji Oil Co., Ltd.), 20 parts of thiamine hydrochloride (manufactured by Tokyo Kasei Kogyo Co., Ltd.) and 20 parts of titanium dioxide were dispersed to obtain a core preparation composition (a dispersion for preparing a core).

In a warmed solution of 70 parts of extremely hardened oil of rice (melting point 53°C (52 to 54°C), Boso oil and fat Co., Ltd.), 30 parts of titanium dioxide was dispersed to obtain an inner shell film preparation composition including an oily substance constituting the inner shell film.

As a shell film composition used to form an outer shell film, 15 parts of carrageenan (manufactured by Sansho Co., Ltd.), 50.9 parts of dextrin (manufactured by Nippon Starch Chemical Co., Ltd.; DE value of less than 10), 3 parts of sorbitol (manufactured by Mitsubishi Shoji Foodtech Co., Ltd.), 10 parts of pectin (manufactured by Unitec Foods Co., Ltd.), 1 part of calcium chloride and 0.1 parts of calcium chloride were dissolved in 400 parts of purified water, and then 10 parts of titanium dioxide was dispersed therein to prepare the shell film composition.

A seamless capsule of a three-layered structure having an average diameter of 6 mm was prepared by:
ejecting the core preparation composition from a first nozzle (innermost nozzle) of a concentric triple nozzle, ejecting the inner shell film preparation composition from a second nozzle on the outside of the first nozzle (intermediate nozzle) and ejecting the shell film composition from a third nozzle of the concentric triple nozzle (outermost nozzle) simultaneously into rapeseed oil flowing at normal temperature (20°C) under the conditions of a concentric nozzle temperature of 50°C to form a composite jet; and
releasing the composite jet into an oil solution. Dried seamless capsules were obtained by normal throughflow drying of the resulting capsules.

The resulting dried seamless capsules were classified by using JIS test sieves (JIS Z 8801-1).

A maximum particle size and minimum particle size of the seamless capsules obtained as described above were determined by performing digital processing with a digital microscope VHX-2000 (KEYENCE Corporation). The results are shown in the following Table 1.

### Examples 3 to 8 and Comparative Examples 1 to 6

In Examples 3 to 8 and Comparative Examples 1 to 6, dried seamless capsules were obtained by the procedure as described in Example 2, except that composition ratios of the core, the intermediate layer and the shell film were changed as shown in Table 1.

**Table 1-1**

| | Example | | | | | | |
|---|---|---|---|---|---|---|---|
| | 2 | 3 | 4 | 5 | 6 | 7 | 8 |
| | | | | | | | |
| Thiamine hydrochloride | 20 | 20 | 20 | 20 | 20 | 20 | |
| L-ascorbic acid | | | | | | | 20 |
| Melano H1000S | 60 | | | 50 | | | |
| Melano H1000 | | 50 | | | 60 | | |
| Melano H3000 | | | 60 | | | 50 | 60 |
| Permel 45 | | | | | | | |
| Titanium dioxide | 20 | | 20 | | 20 | | 20 |
| Talc | | 30 | | 30 | | 30 | |
| Calcium carbonate | | | | | | | |
| | | | | | | | |
| Extremely hardened oil of rice | 70 | 60 | 70 | 70 | 80 | 70 | 70 |
| Titanium dioxide | 30 | | 30 | | 20 | | 30 |
| Talc | | 40 | | 30 | | 30 | |
| | | | | | | | |
| Carrageenan | 15 | 15 | 15 | 15 | 15 | 15 | 15 |
| Pectin | 10 | 10 | 10 | 10 | 10 | 10 | 10 |
| Dextrin | 50.9 | 45.9 | 50.9 | 50.9 | 50.9 | 45.9 | 50.9 |
| KCl | 1 | 1 | 1 | 1 | 1 | 1 | 1 |
| CaCl₂ | 0.1 | 0.1 | 0.1 | 0.1 | 0.1 | 0.1 | 0.1 |
| Sorbitol | 13 | 13 | 13 | 13 | 13 | 13 | 13 |
| Titanium dioxide | 10 | | 10 | 10 | 10 | | 10 |
| Talc | | 15 | | | | 15 | |
| Distilled water | 400 | 400 | 400 | 400 | 400 | 400 | 400 |
| Specific gravity | 1.33 | 1.33 | 1.33 | 1.29 | 1.29 | 1.29 | 1.33 |
| Breaking strength (N) | 0.5 | 0.9 | 4.7 | 0.5 | 0.9 | 4.7 | 4.7 |
| Maximum particle size (mm) | 7.47 | 7.47 | 7.47 | 7.47 | 7.47 | 7.47 | 7.47 |
| Minimum particle size (mm) | 5.76 | 5.76 | 5.76 | 5.76 | 5.76 | 5.76 | 5.76 |

**Table 1-2**

| | Comparative Example | | | | | |
|---|---|---|---|---|---|---|
| | 1 | 2 | 3 | 4 | 5 | 6 |
| | | | | | | |
| Thiamine hydrochloride | 20 | 20 | 20 | 20 | 20 | 20 |
| L-ascorbic acid | | | | | | |
| Melano H1000S | | | 80 | | | |
| Melano H1000 | | | | 80 | | |
| Melano H3000 | | | | | 80 | |
| Permel 45 | 50 | 60 | | | | 80 |
| Titanium dioxide | | 20 | | | | |
| Talc | 30 | | | | | |
| Calcium carbonate | | | 10 | 10 | 10 | 10 |
| | | | | | | |
| Extremely hardened oil of rice | 60 | 80 | 100 | 100 | 100 | 100 |
| Titanium dioxide | | 20 | | | | |
| Talc | 40 | | | | | |
| | | | | | | |
| Carrageenan | 15 | 15 | 15 | 15 | 15 | 15 |
| Pectin | 10 | 10 | 10 | 10 | 10 | 10 |
| Dextrin | 45.9 | 50.9 | 60.9 | 60.9 | 60.9 | 60.9 |
| KCl | 1 | 1 | 1 | 1 | 1 | 1 |
| CaCl₂ | 0.1 | 0.1 | 0.1 | 0.1 | 0.1 | 0.1 |
| Sorbitol | 13 | 13 | 13 | 13 | 13 | 13 |
| Titanium dioxide | | 10 | | | | |
| Talc | 15 | | | | | |
| Distilled water | 400 | 400 | 400 | 400 | 400 | 400 |
| Specific gravity | 1.33 | 1.29 | 1.16 | 1.16 | 1.16 | 1.16 |
| Breaking strength (N) | 5.4 | 5.4 | 0.5 | 0.9 | 4.8 | 5.4 |
| Maximum particle size (mm) | 7.47 | 7.47 | 7.47 | 7.47 | 7.47 | 7.47 |
| Minimum particle size (mm) | 5.76 | 5.76 | 5.76 | 5.76 | 5.76 | 5.76 |

L-ascorbic acid: Wako Pure Chemical Industries, Ltd.
Melano H1000: Hydrogenated palm kernel oil from Fuji Oil Co., Ltd., melting point 38°C (36 to 40°C)
Melano H3000: Hydrogenated palm kernel oil from Fuji Oil Co., Ltd., melting point 42°C (40 to 44°C)
Permel 45: Intermediate melting point fraction of palm fraction oil from Fuji oil Co., Ltd., melting point 45°C (43 to 47°C)

With respect to each of the dried seamless capsules obtained, a specific gravity of the seamless capsule was determined using isooctane as a specific gravity standard solution, based on the Archimedes method by:
putting 5 mL of isooctane in a 10 mL-measuring cylinder,
putting ten seamless capsules therein,
reading the volume of isooctane increased from 5 mL, and
determining a product of the volume and specific gravity of isooctane. The step was repeated 10 times, and the average value thereof was shown in Table 1 as a value of the specific gravity of the resulting seamless capsules.

With respect to each of the dried seamless capsules obtained, a breaking strength (N) of a portion having the minimum particle size thereof was determined using a comprehensive physical property measurement device (Sun Scientific Co., Ltd, rheometer) by:
pressing a circular plunger against the seamless capsule (at a table speed of 20 mm/min) under the environment of 39°C, which is almost the same as body temperature of cattle, and
measuring a load required to break the seamless capsule. An average value of the breaking strength measured with respect to 20 capsules of the same specifications is shown in Table 1 as a breaking strength.

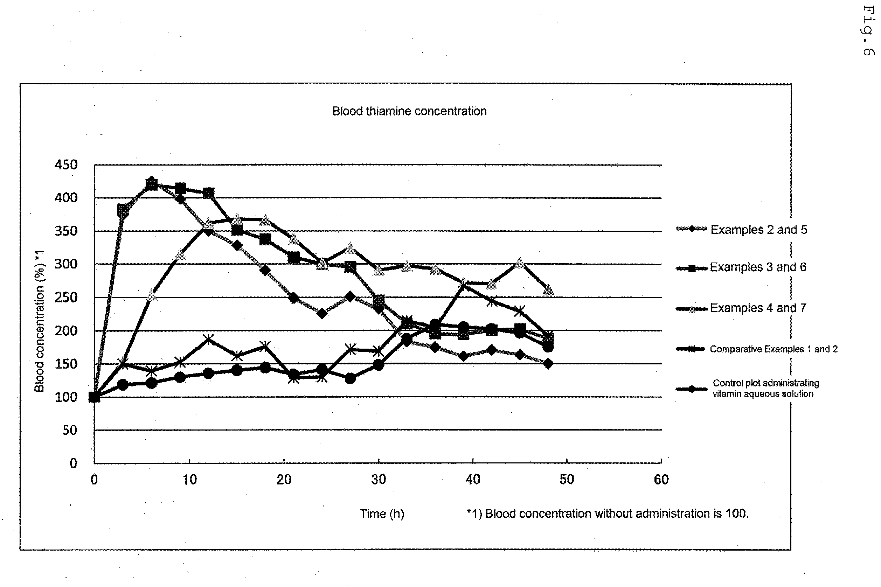

The resulting capsules of Examples 2 to 7 and Comparative Examples 1 to 6 were orally administered to six adult cattle in an amount such that vitamin C is 30g in a forced manner by using an oral administration device. Then, blood was collected from jugular vein with time every three hours, 1800 µL of 5%-trichloroacetic acid (formed by dissolving 10 g of trichloroacetic acid in 100 mL of ultra-pure water and diluting with ultra-pure water in a measuring cylinder to 200 mL total) was added to 900 µL of whole blood, and the mixture solution was centrifuged at 14,000×g, for 5 minutes (4°C). The resulting supernatant of about 1.5 mL was filtered through a 0.45 µm filter and was transferred to a vial to obtain a measurement sample. Thereafter, time-dependent change of blood thiamine concentration was measured by a high-performance liquid chromatography (HPLC) under the conditions shown in Table 2. The results are described in Fig.6 as a graph using the time as the abscissa and the blood thiamine concentration as the ordinate.

**Table 2**

| HPLC condition | |
|---|---|
| Column: | COSMOSIL 5C18-MC-II (4.6 mm I.D. × 250mm, Nacalai Tesque Inc.), |
| | Column temperature 45°C |
| Eluent (Mobile phase) | 0.2 M Sodium dihydrogenphosphate aq/ 0.3%(v/v) Acetonitrile aq (997/3), |
| | Flow rate 1.0 mL/min |
| Sample injection amount | 350 µL |
| Reaction solution (1): | 0.01%(w/v) Potassium ferricyanide, |
| | Flow rate 0.5 mL/min |
| Reaction solution (2): | 15%(w/v) Sodium hydroxide |
| | Flow rate 0.5 mL/min |
| Reaction coil: | PEEK tube (0.5 mm I.D. × 1200 mm) |
| | Reaction temperature 45°C |
| Detector: | Fluorescence detector (Hitachi High-Technologies Corporation) |
| | Ex. 375nm, Em. 450nm |

As a control plot, with respect to 5 adult cattle which were made to drink an aqueous solution such that the thiamine hydrochloride in the same amount as the administered amount was dissolved in water, the same measurement was performed.

With respect to the capsules of Comparative Examples 3 to 6, cattle rumen fluid collected was shaken for 24 hours at 39°C in a flask, and flotation was observed. Since it is unlikely to avoid the rumination, it was not adopted for an administration test.

Since the flotation was not observed in the above study with respect to Examples 2 to 8 and Comparative Examples 1 and 2, the administration test was performed with respect to the capsules of Examples 2 to 7 and Comparative Examples 1 and 2 including vitamin B1. The results of the administration test are shown in Fig.6.

As is apparent from FIG. 6, with respect to the capsules, of which the breaking strength of a portion having the minimum particle size is within the range of 0.5 to 4.7 N, there was a major change in blood thiamine concentration in an initial stage of the measurement (after about 2 hours) as long as it satisfies the specific gravity, and it was indicated that administration of the vitamin effectively acted thereon. On the other hand, with respect to Comparative Examples 1 to 2 and the control, there was a minor change, but it was not recognized that it quickly responded to the administration of the vitamin.

### Comparison with commercial products

In order to compare Vitamin C 30% Bypass (YPTECH Co., Ltd.; Hereinafter, it is indicated as an existing product.) with the present invention, each of the resulting capsule in Example 8 of Table 1 and the existing product was orally administered to six lactating cows in an amount such that vitamin C is 30 g in a forced manner by using an oral administration device. Then, whole urine up to 24 hours was collected, 9 mL of the urine every the sampling time was mixed in a Spitz tube containing 1 mL of 1 M hydrochloric acid, after the invert, it was dispensed to two cryo-tubes (for analysis and storage), and then cryo-preserved (VC common sample). The urine dissolved of about 1.5 mL was filtered through a 0.45 µm filter and was transferred to a vial to obtain a measurement sample. Thereafter, HPLC determination was performed under the following conditions.

As a control plot, with respect to six lactating cows which were made to drink an aqueous solution such that the vitamin C in the same amount as the administered amount was dissolved in water, the same measurement was performed.

### Conditions

Column: Tosoh ODS 120T (Average particle diameter 5 um, 4.6 mm I.D. × 150 mm, manufactured by Tosoh Corporation),
Column temperature: 35°C
Eluent (mobile phase): 10 mM ammonium formate aq/ 70 mM dodecyltrimethylammonium bromide aq/ water/ methanol (1/1/4/4),
Flow rate: 0.9 mL/ min.
Sample injection amount: 10 µL
Detector: Ultraviolet-visible detector (manufactured by Hitachi High-Technologies Corporation) 265 nm

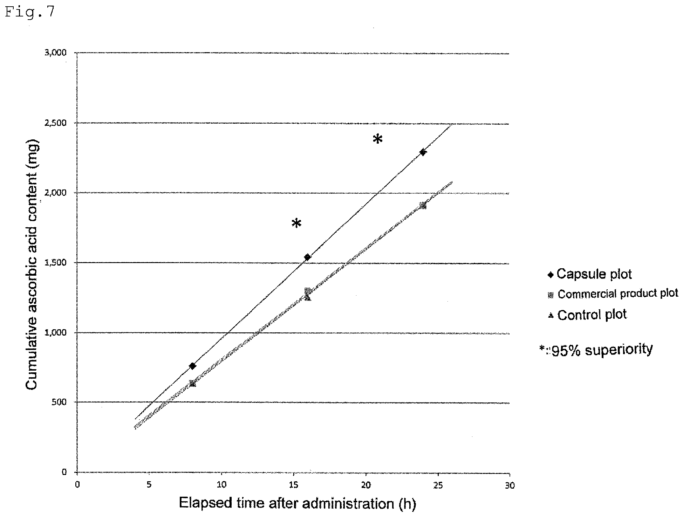

Fig.7 is a graph illustrating cumulative excretion of urinary ascorbic acid (integrated ascorbic acid amount) measured using the measurement sample. It is shown in the graph.

With respect to the existing product, a particle size, specific gravity and breaking strength of a portion having the minimum particle size were measured, and as the result, the particle size was 1.0 mm, the specific gravity was 1.12 and the breaking strength of a portion having the minimum particle size was 2.0 N. Here, in the specific gravity measurement of the existing product, pure water was used as a specific gravity standard solution.

As is apparent from Fig.7, the cumulative excretion of vitamin C was remarkably increased in the case of using the seamless capsule of Example 8. On the other hand, the cumulative excretion of vitamin C was approximately the same as the control plot in the case of using the existing product.

### Example 9

### Production of seamless capsule

Seamless capsules containing a freeze-dried powder of lactic acid bacteria (*Lactobacillus coryniformis* JCM 1099) and starch as a core of the seamless capsules were produced.

Ten parts of lyophilized L. coryniformis product (6.9 × 10¹⁰ cfu/g), 10 parts of starch, and 20 parts of titanium dioxide were dispersed in a warmed solution of 60 parts of Melano H3000 (Fuji Oil Co., Ltd.; hydrogenated palm kernel oil having a melting point of 42°C (40 to 44°C)) to obtain a core preparation composition (a dispersion for preparing a core).

A dried seamless capsule was obtained as described in Example 4, except that the core preparation composition obtained as described above was used.

With respect to the resulting dried seamless capsule, the maximum particle size, the minimum particle size, the breaking strength and the specific gravity were determined as described in Example 1. The results are as follows.
Maximum particle size: 7.47 mm
Minimum particle size: 5.76 mm
Breaking strength: 4.7 N
Specific gravity: 1.33

### Oral administration to adult milk cow

The seamless capsules obtained as described above were orally administered to two adult lactating cows in an amount such that a number of living bacteria in lactic acid bacteria is 3.0 × 10¹¹ cfu per one cow in a forced manner by using an oral administration device once a day at a fixed time for seven days. The feces were collected from the rectum 24 hours after every administration, and DNA was extracted by using PowerSoil DNA Isolation Kit (MO BIO Laboratories, Inc.). With respect to a region encoding 16S rRNA of Lactobacillus, PCR was performed using the following primers, and amplified product was fractionated by a DGGE (Denaturing Gradient Gel Electrophoresis) method.
f: (5'- GTC GTC AGC TCG TGT CGT GAG A -3'),
r: (5'- CGC CCG CCG CGC CCC GCG CCC GGC CCG CCG CCC CCG CCC CCC CGG GAA CGT ATT CAC CGC -3')
   A region obtained by the fractionation described above was extracted with Qiaex II gel extraction kit (Qiagen), with respect to a region encoding 16S rRNA of L. *coryniformis* spp, PCR was performed further using the following primers.
f: (5'- GGG TTC GCA CGA GCG CAC -3')
r: (5'- CGC CCG CCG CGC CCC GCG CCC GGC CCG CCG CCC CCG CCC CCC CGG GAA CGT ATT CAC CGC -3')

A product amplified by a PCR method was fractionated by a DGGE method.

As a result, in electrophoretic patterns of DNA extracted from feces collected after 24 hours from the administration of the sixth day, a band matching the DNA of administered lactic acid bacteria (*L. coryniformis* JCM 1099) was observed. Therefore, it was confirmed that the capsule was not disintegrated in the rumen to abomasum of the adult milk cow; and was disintegrated in and after intestine to release the lactic acid bacteria from the capsule.

### INDUSTRIAL APPLICABILITY

According to the orally administered agent for ruminants of the present invention, a drug may reach lower digestive tract of the ruminants, and may allow to be absorbed therein only by including the orally administered agent in ruminant feed and the like. By using the orally administered agent for ruminants of the present invention, it is possible to very simply administer the drug to ruminants. The present invention also provides a ruminant feed containing the orally administered agent for ruminants. Further, according to the present invention, by administering the orally administered agent to the ruminants, it is possible to treat diseases of ruminants, increase the digestibility, or administer vitamins thereto.

## Claims

1. An orally administered agent for ruminants which reaches lower digestive tract without decomposition in the rumen wherein a specific gravity determined using isooctane as a specific gravity standard solution is within the range of 1.17 to 2.00, a maximum particle size is within the range of 4 to 10 mm and a minimum particle size is within the range of 4 to 10 mm, and a breaking strength of a portion having the minimum particle size under body temperature environment of the ruminants is within the range of 0.5 to 5.0 N.

2. The orally administered agent for ruminants according to Claim 1, wherein the orally administered agent is a seamless capsule comprising a core, and at least one layer of shell film layer covering the core, and wherein the core comprises a carrier and a drug for ruminants.

3. The orally administered agent for ruminants according to Claim 2,
wherein the shell film layer is comprised of two layers of an inner shell film in direct contact with the core and an outer shell film as an outermost layer,
wherein the core further comprises a specific gravity regulator,
wherein the inner shell film comprises a specific gravity regulator, and an oily substance having a melting point of 45 to 90°C, and
wherein the outer shell film is formed from a shell film composition comprising polysaccharide, a specific gravity regulator, and a plasticizer.

4. The orally administered agent for ruminants according to Claim 2 or 3, wherein the core has a melting point of 32 to 44°C.

5. The orally administered agent for ruminants according to any one of Claims 2 to 4, wherein the drug for ruminants is selected from the group consisting of herbal medicine extract, tincture, a therapeutic agent, plant extract, animal extract, microorganism extract, microbial production extract, fruit juice, functional polysaccharides, polyphenols, vitamin C, vitamin B, amino acids, microorganisms, bacteria, essential oil, ω-3 fatty acid, ω-6 fatty acid, ω-9 fatty acid and combinations thereof.

6. A method of making the orally administered agent for ruminants as defined in any one of claims 1 to 5, said method comprising the steps of:
by using a first nozzle, a second nozzle and a third nozzle such that they are concentrically arranged and have a radius sequentially increased from the first nozzle to the third nozzle, ejecting a core preparation composition through the first nozzle, ejecting an inner shell film preparation composition through the second nozzle and ejecting a shell film composition through the third nozzle simultaneously to form a composite jet, and
releasing the composite jet into an oil solution,
wherein the core preparation composition comprises a specific gravity regulator, a carrier and a drug for ruminants,
wherein the inner shell film preparation composition comprises a specific gravity regulator, and an oily substance having a melting point of 45 to 90°C, and
wherein the shell film composition comprises polysaccharide, a specific gravity regulator, and a plasticizer.

7. A ruminant feed comprising the orally administered agent for ruminants according to any one of Claims 1 to 5.

## Patentansprüche

1. Oral zu verabreichendes Mittel für Wiederkäuer, das den unteren Verdauungstrakt ohne Zersetzung im Pansen erreicht, wobei die unter Verwendung von Isooctan als Standardlösung für die relative Dichte bestimmte relative Dichte im Bereich von 1,17 bis 2,00 liegt, die maximale Teilchengröße im Bereich von 4 bis 10 mm liegt und die minimale Teilchengröße im Bereich von 4 bis 10 mm liegt und die Bruchfestigkeit eines Teils, das die minimale Teilchengröße aufweist, unter Körpertemperaturumgebung der Wiederkäuer im Bereich von 0,5 bis 5,0 N liegt.

2. Oral zu verabreichendes Mittel für Wiederkäuer gemäß Anspruch 1, wobei das oral zu verabreichende Mittel eine nahtlose Kapsel ist, die einen Kern und wenigstens eine Schicht einer Hüllfilmschicht, die den Kern bedeckt, umfasst und wobei der Kern einen Träger und einen Wirkstoff für Wiederkäuer umfasst.

3. Oral zu verabreichendes Mittel für Wiederkäuer gemäß Anspruch 2, wobei die Hüllfilmschicht aus zwei Schichten besteht, einem inneren Hüllfilm in direktem Kontakt mit dem Kern und einem äußeren Hüllfilm als äußerster Schicht,
wobei der Kern weiterhin einen Dichteregulator umfasst,
wobei der innere Hüllfilm einen Dichteregulator und eine ölige Substanz mit einem Schmelzpunkt von 45 bis 90 °C umfasst und
wobei der äußere Hüllfilm aus einer Hüllfilmzusammensetzung gebildet wird, die Polysaccharid, einen Dichteregulator und einen Weichmacher umfasst.

4. Oral zu verabreichendes Mittel für Wiederkäuer gemäß Anspruch 2 oder 3, wobei der Kern einen Schmelzpunkt von 32 bis 44 °C aufweist.

5. Oral zu verabreichendes Mittel für Wiederkäuer gemäß einem der Ansprüche 2 bis 4, wobei der Wirkstoff für Wiederkäuer aus der Gruppe ausgewählt ist, die aus einem Pflanzenmedizinextrakt, einer Tinktur, einem therapeutischen Mittel, einem Pflanzenextrakt, einem Tierextrakt, einem Mikroorganismusextrakt, einem Extrakt aus mikrobieller Produktion, einem Fruchtsaft, funktionellen Polysacchariden, Polyphenolen, Vitamin C, Vitamin B, Aminosäuren, Mikroorganismen, Bakterien, ätherischem Öl, ω-3-Fettsäure, ω-6-Fettsäure, ω-9-Fettsäure und Kombinationen davon besteht.

6. Verfahren zur Herstellung des oral zu verabreichenden Mittels für Wiederkäuer gemäß einem der Ansprüche 1 bis 5, wobei das Verfahren die folgenden Schritte umfasst:
unter Verwendung einer ersten Düse, einer zweiten Düse und einer dritten Düse, die konzentrisch angeordnet sind und einen von der ersten Düse zur dritten Düse sukzessive zunehmenden Radius aufweisen, gleichzeitiges Austragen einer Kernpräparatzusammensetzung durch die erste Düse, einer innerer-Hüllfilm-Präparatzusammensetzung durch die zweite Düse und einer Hüllfilmzusammensetzung durch die dritte Düse unter Bildung eines zusammengesetzten Strahls und
Freisetzen des zusammengesetzten Strahls in eine Öllösung,
wobei die Kernpräparatzusammensetzung einen Dichteregulator, einen Träger und einen Wirkstoff für Wiederkäuer umfasst,
wobei die innerer-Hüllfilm-Präparatzusammensetzung einen Dichteregulator und eine ölige Substanz mit einem Schmelzpunkt von 45 bis 90 °C umfasst und
wobei die Hüllfilmzusammensetzung Polysaccharid, einen Dichteregulator und einen Weichmacher umfasst.

7. Wiederkäuerfutter, das das oral zu verabreichende Mittel für Wiederkäuer gemäß einem der Ansprüche 1 bis 5 umfasst.

## Revendications

1. Agent administré par voie orale destiné à des ruminants, qui atteint le tube digestif inférieur sans décomposition dans le rumen, dans lequel une densité déterminée en utilisant de l'isooctane comme solution étalon de densité se situe dans la plage de 1,17 à 2,00, une taille de particule maximum se situe dans la plage de 4 à 10 mm et une taille de particule minimum se situe dans la plage de 4 à 10 mm, et une résistance à la rupture d'une partie ayant la taille de particule minimum dans l'environnement de température corporelle des ruminants se situe dans la plage de 0,5 à 5,0 N.

2. Agent administré par voie orale destiné à des ruminants selon la revendication 1, dans lequel l'agent administré par voie orale est une capsule sans soudure comprenant un noyau, et au moins une couche d'une couche de film d'enveloppe recouvrant le noyau, le noyau comprenant un support et un médicament destiné à des ruminants.

3. Agent administré par voie orale destiné à des ruminants selon la revendication 2,
dans lequel la couche de film d'enveloppe comprend deux couches d'un film d'enveloppe interne en contact direct avec le noyau et d'un film d'enveloppe externe en tant que couche la plus externe,
dans lequel le noyau comprend en outre un régulateur de densité,
dans lequel le film d'enveloppe interne comprend un régulateur de densité, et une substance huileuse ayant un point de fusion de 45 à 90°C, et
dans lequel le film d'enveloppe externe est formé à partir d'une composition de film d'enveloppe comprenant un polysaccharide, un régulateur de densité et un plastifiant.

4. Agent administré par voie orale destiné à des ruminants selon la revendication 2 ou 3, dans lequel le noyau a un point de fusion de 32 à 44°C.

5. Agent administré par voie orale pour ruminants selon l'une quelconque des revendications 2 à 4, dans lequel le médicament destiné à des ruminants est choisi dans le groupe consistant en un extrait de phytothérapie, une teinture, un agent thérapeutique, un extrait de plante, un extrait animal, un extrait de microorganisme, un extrait de production microbienne, un jus de fruits, des polysaccharides fonctionnels, des polyphénols, de la vitamine C, de la vitamine B, des acides aminés, des microorganismes, des bactéries, de l'huile essentielle, de l'acide gras ω-3, de l'acide gras ω-6, de l'acide gras ω-9 et des combinaisons de ceux-ci.

6. Procédé de fabrication de l'agent administré par voie orale destiné à des ruminants tel que défini dans l'une quelconque des revendications 1 à 5, ledit procédé comprenant les étapes consistant à :
utiliser une première buse, une deuxième buse et une troisième buse de telle sorte qu'elles sont agencées de façon concentrique et ont un rayon accru séquentiellement à partir de la première buse vers la troisième buse, l'éjection d'une composition de préparation de noyau à travers la première buse, l'éjection d'une composition de préparation de film d'enveloppe interne à travers la deuxième buse et l'éjection d'une composition de film d'enveloppe à travers la troisième buse simultanément pour former un jet composite, et
libérer le jet composite dans une solution d'huile,
dans lequel la composition de préparation de noyau comprend un régulateur de densité, un support et un médicament destiné à des ruminants,
dans lequel la composition de préparation de film d'enveloppe interne comprend un régulateur de densité et une substance huileuse ayant un point de fusion de 45 à 90°C, et
dans lequel la composition de film d'enveloppe comprend un polysaccharide, un régulateur de densité et un plastifiant.

7. Aliment destiné à des ruminants comprenant l'agent administré par voie orale destiné à des ruminants selon l'une quelconque des revendications 1 à 5.
